# EUROPEAN PATENT APPLICATION

(11) **EP 0 887 641 A1**
(43) Date of publication of application: **30.12.1998**
(21) Application number: 98304929.7
(22) Date of filing: 23.06.1998
(51) Int. Cl.: G01N 27/49, G01N 27/416

(54) **Electrochemical sensor for detection of chlorine in phosgene**

(30) Priority: 23.06.1997 US 880959
(71) Applicant: GENERAL ELECTRIC COMPANY, Schenectady, NY 12345 (US)
(72) Inventor: Gui, John Yupeng, Niskayuna, New York 12309 (US); Barren, Elizabeth, Scotia, New York 12302 (US)
(74) Representative: Goode, Ian Roy

(57) **Abstract**

An online system for detecting the presence of low levels of chlorine in a stream of phosgene is described. Detection is based on the electrochemical reduction of chlorine at a platinum electrode at potentials at which other components of the phosgene stream and electrolyte cause no interference. A continuous flow of electrolyte over the surface is maintained by gravity feed or by positive pumping mechanism (34). The electrolyte flow rate is about 1 to about 10ml per hour. Organic electrolytes are preferred although aqueous electrolytes can be used. The sensor (30) of this invention can detect very low levels of chlorine. The electrode can be regenerated in place. The detector system comprises five main components including a gas delivery subsystem (2, 4, 6), the electrochemical sensor (30), a temperature control unit (42), a signal measurement subsystem (50, 52, 54), and a scrubber subsystem.

## Description

This invention is directed to an electrochemical sensor system for detecting and measuring chlorine content in phosgene and more particularly to a sensor for monitoring trace levels of chlorine in a phosgene process stream.

Carbonyl chloride or phosgene is used in the production of polycarbonate polymers by the direct phosgenation of a bisphenol such as bisphenol A. The presence of even small amounts of chlorine in the phosgene can cause serious quality problems in the polycarbonate manufacturing process and in the polycarbonate resin. Formation of colored reaction species in the resin is one example.

Online detection of chlorine in phosgene is complicated by toxicity and corrosiveness of both materials. UV absorbance based on the spectral difference between chlorine and phosgene is available, but sensitivity and reliability are unsatisfactory. Commercial electrochemical sensors for chlorine monitoring, available for occasional leak detection, are not capable of continuous operation due to limited electrolyte supply and to degradation of electrode surfaces in the gas stream.

This invention is an online system for detecting the presence of low levels of chlorine in a stream of phosgene. Detection is based on the electrochemical reduction of chlorine at a platinum electrode at potentials at which other components of the phosgene stream and electrolyte cause no significant interference. To ensure long-term consistency of the electrode a continuous flow of electrolyte over the surface is maintained by gravity feed or by a positive pumping mechanism. The electrolyte flow rate is about 1 to about 10 ml per hour. Variation of the flow rate has no significant effect on the sensitivity of the sensor electrode. Organic electrolytes are preferred although aqueous electrolytes can be used. The sensor of this invention can detect very low levels of chlorine, e.g., from less than 1 ppm to several percent. The electrode can be regenerated in place.

The detector system comprises five main components including a gas delivery subsystem, the electrochemical sensor, a temperature control unit, a signal measurement subsystem, and

An embodiment of the invention will now be described, by way of example, with reference to the accompanying drawings, in which:
Figure 1 is a schematic view of the overall detector system.
Figure 2 is a sectional view of an electrochemical sensor cell and associated components.

The system comprises three gas lines phosgene, nitrogen, and chlorine in nitrogen. Each gas line is provided with a flow controller and metering device. We have experimented with two types of such devices. Suitable flow controllers include mass flow controllers and a flowmeter with a needle valve. ( Although a mass flow controller deliveries are more accurate and persistent gas flow than a flowmeter, it may need frequent maintenance due to orifice blockage caused by corrosion or the deposition of particulates from the gas stream. A flowmeter requires virtually no maintenance and meets the flow accuracy requirements for most of on-line measurements.

The gas delivery system is housed inside an air-tight enclosure which provides both air isolation for protecting operators in case of phosgene leakage from phosgene lines and thermal isolation to avoid phosgene condensation at low temperatures. All gas flows are controlled by appropriate needle valves mounted on the top of the box and measured by the flowmeters. Nitrogen can be used to form a chlorine standard for calibration and also for flushing the sensor system. Stainless steel and Teflon tubing are suitable for all line connections. Check valves can be used to avoid cross contamination between the gas lines.

The electrochemical sensor system comprises an electrochemical cell with at least two electrodes, a potentiostat, and an electrolyte delivery apparatus. The most important component of the sensor is the platinum (Pt) sensing electrode, also called the working electrode. Chlorine is electrochemically reduced to chlorine anion (Cl⁻ ) at the working electrode and the resulting reduction current that passes through the working electrode is recorded on a measuring device. The higher the chlorine concentration in the gas stream, the higher the measured current. The other electrode is an auxiliary electrode, also termed a counter electrode. A piece of silver wire is used. When chlorine is reduced at the working electrode, the silver auxiliary electrode is simultaneously oxidized to silver chloride (AgCl) in an electrolyte containing chlorine anions. The net cell reaction is chlorine oxidizing silver to form silver chloride and no soluble by-product is generated. The third electrode is a reference electrode the main function of which is to allow a potentiostat to provide a constant potential for the working electrode. The reference electrode is a silver/silver chloride electrode which can be made by simply inserting a piece of silver wire into an electrolyte containing Cl⁻ anions to form a thin layer of silver chloride at the silver surface. It is possible to combine the auxiliary and reference electrodes into one. However, it is more advantageous to use separate electrodes.

To ensure consistent surface properties of the Pt sensor electrode, a continuous flow of electrolyte over the Pt surface was used. Using renewable electrolytes significantly improves the sensor's stability, reproducibility, and operation life time. Since the electrolyte flows continuously over the sensor electrode, the electrode surface and the electrolyte in the electrode double-layer are periodically renewed. This continuous electrolyte flow is achieved through an electrolyte delivery system consisting of a pump and a Teflon check valve. The pump is used not only for controlling the electrolyte flow, but also for providing positive electrolyte pressure to avoid possible phosgene breakthrough through the porous material. The purpose of the Teflon check valve is to avoid accidental phosgene breakthrough should a leak occur in the electrolyte delivery system, (e.g. a break in the peristaltic pump tubing). Both aqueous and non-aqueous electrolytes have been employed successfully. The main advantage of an aqueous solution is its low cost; a disadvantage is that phosgene can hydrolyze and form hydrogen chloride which creates a corrosive environment.

The chlorine sensor system is isolated in a thermally controlled enclosure or box. The box serves two purposes. It maintains the sensor at a constant temperature to prevent phosgene from condensing at low temperatures (<15°C). It also prevents release of phosgene in the event of a leak from the sensor. Heat can be provided to the enclosure by any convenient means. The heat source can be operated by a temperature controller. To avoid overheating of the box due to a malfunctioning thermal coupler, a second temperature controller can be installed in series with the primary temperature controller.

The sensor current can be measured by several methods: (1) open-circuit potential with a voltameter, (2) close-circuit current measurement with an amperometer, (3) voltage drop measurement by passing the current through a load resistor (<100 Ohms), or (4) voltage measurement using a current-to-voltage converter based on an operation amplifier circuit. The last measurement method is preferred because it does not experience external IR drop problems. The current-to-voltage converter is an integrated circuit of a potentiostat, and it is easy for both analog and digital recording. To meet manufacturing plant standard for signal transmission in the 4-20 mA range, a configurable isolator which will convert voltage signal from the current-to-voltage converter back to 4-20 mA current signal can be added to the system.

In a laboratory test condition, caustic solutions were used to scrub both phosgene and chlorine. To avoid possible phosgene break through, a two stage scrubber system was used. The first stage scrubber solution was made from 45% concentrated aqueous KOH solution, water, and methanol in the volume ratio of 1:2:3; the second stage scrubber solution was prepared from a 45% concentrated aqueous KOH solution and methanol in the volume ratio of 1:2. Phenol Red was used as a pH indicator for the scrubber solutions. Disappearance of the red color indicated that the scrubber solution was spent. It is important to prevent clogging of the phosgene vent line by salt (K₂CO₃) build-up at the solution interface, particularly in the first scrubber solution. It was helpful to utilize a V-shaped connector with its small end connected to the phosgene vent tubing (1/4" OD Teflon tubing) and its large diameter (about 1" ID) end immersed about 1.5 inches below the surface of the scubber solution. In a plant operating environment, the phosgene can be vented to a scrubber tower.

There are several ingredients in an electrolyte solution: solvents, electrolyte salts, special reagents and pH buffer. The electrolyte solution has to contain a component which will form an insoluble product with silver ion at the silver auxiliary electrode; not be reduced electrochemically at the platinum working electrode; and not react chemically with chlorine. Chlorine anion (Cl⁻)is the preferred choice.

There are two major types of solvent systems for the electrolyte, aqueous based and organic solvent systems. When water is used as a solvent, the salts used to make an electrolyte solution are typically inorganic or very polar organic salts because of their large solubilities in water. Such salt-water solutions are called aqueous electrolyte solutions. Metal chlorides such as lithium chloride, sodium chloride and potassium chloride can be used at concentrations can range from about 1 millimolar to saturation. A concentration in the range of about 0.1 to about 1 molar provides a good balance of solution conductivity and avoidance of excess salt precipitation. When an organic compound is used as a solvent, the salts used are those organic salts which have sufficient solubility in the organic solvent. This salt-organic solution is termed an organic electrolyte solution. Suitable organic solvents include acetonitrile (AN), propylene carbonate (PC), dimethyl sulfoxide (DMSO), and dimethylformamide (DMF). Other polar organics can also be used as electrolyte solvents provided they have a moderately high dielectric constant, e.g., greater than about 10, and do not interfere with chlorine detection. Suitable organic salts include (tetrabutylammonium chloride (TBACI), tetrabutylammonium hexafluorophosphate (TBAHFP), tetrabutylammonium tetrafluoroborate (TBATFB), and other related alkylammonium anion salts, including combinations of such salts. A mixture of tetrabutylammonium chloride and tetrabutylammonium hexafluorophosphate provides good performance as the electrolyte salts. Tetrabutylammonium chloride is a source of chloride anions which are needed for operation of the sensor. However, its solubility is very limited. To increase the conductivity of the electrolyte solution, other organic salts are added to the electrolyte composition. Organic electrolyte solutions are preferred despite the somewhat higher cost. One limitation of aqueous electrolyte solutions is that phosgene reacts with water to form hydrochloric acid and carbon dioxide. Organic electrolytes produce higher electrode currents than aqueous electrolytes for the same chlorine concentration. One reason why an organic electrolyte produces a higher response may be that there is more dispropotionation of chlorine in aqueous media. This effectively lowers the amount of chlorine that is electrochemically reduced.

Although continuous electrolyte flow has significantly extended the electrochemical sensor's operating life, the sensor can fail to operate normally after a long period of usage. The silver auxiliary electrode may accumulate a thick layer of silver chloride film on the electrode surface, thus limiting the current output. The platinum sensoring electrode may undergo a cumulative poisoning effect caused by the presence of adsorbed material on the platinum surface.. the electrolyte flow can be reduced as the fritted glass becomes blocked. The first two conditions can be overcome by in-situ electrode regeneration. In the normal sensor operation, chlorine reduction takes place at the platinum electrode while the silver electrode is oxidized and forms a silver chloride film. During regeneration, the electrochemical processes are reversed. Oxidation will occur at the Pt electrode and reduction will occur at the silver electrode. This is done by applying a high positive potential to the platinum electrode. As the chloride ions are oxidized to elemental chlorine (Cl₂) at the platinum electrode, the silver chloride film on the silver auxiliary electrode is reduced to elemental silver. Most chemical adsorbates (poisons) on the platinum surface can be oxidatively removed. the silver auxiliary electrode will undergo a reduction process whereby the silver chloride I film is oxidized to elemental Ag. Such a reduction process can be visually observed by the color change at the silver surface.

The invention will be more clearly understood when considered in the context of the accompanying drawings.

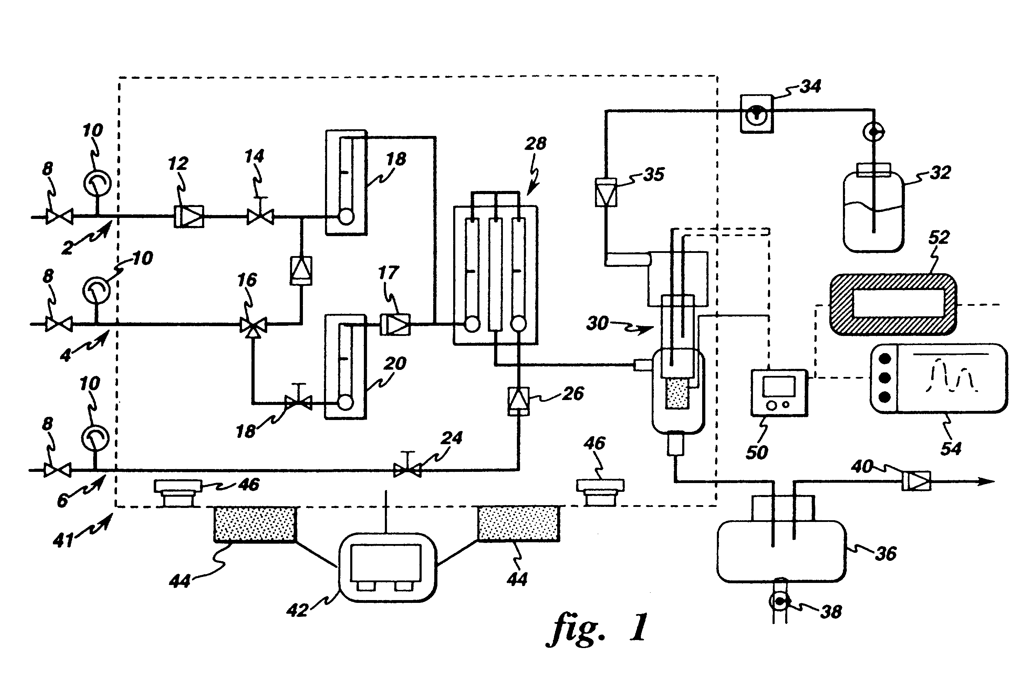

Figure 1 shows in schematic form the overall detection system which comprises a phosgene input line 2, carrier gas line 4, and a chlorine gas line 6, also in a carrier gas calibration purposes. Each of the gas lines is provided with a two-way valve 8 and a pressure gauge 10. The phosgene line 2 is also provided with a check valve, shown at 12, and a needle valve 14. The carrier gas line has a three-way valve 16 and a check valve 17 and needle valve 18. Flow meters 18 and 20 are located on the phosgene and the carrier gas lines. The chlorine and carrier gas line is fitted with a needle valve 24 and a check valve 26. All three gas lines feed into flow meter 28. The output from flow meter 28 is delivered to the electrochemical sensor cell 30 hereafter described in more detail.

Electrolyte for the sensor cell 30 is delivered from reservoir 32 by pump 34 to check valve 35. Electrolyte can also be delivered by gravity flow. The electrolyte flows through sensor cell 30 to waste electrolyte collector 36, which is provided with a drain line and an on off valve 38, and gas vents which leads to the scrubber, which is not shown, via check valve 40.

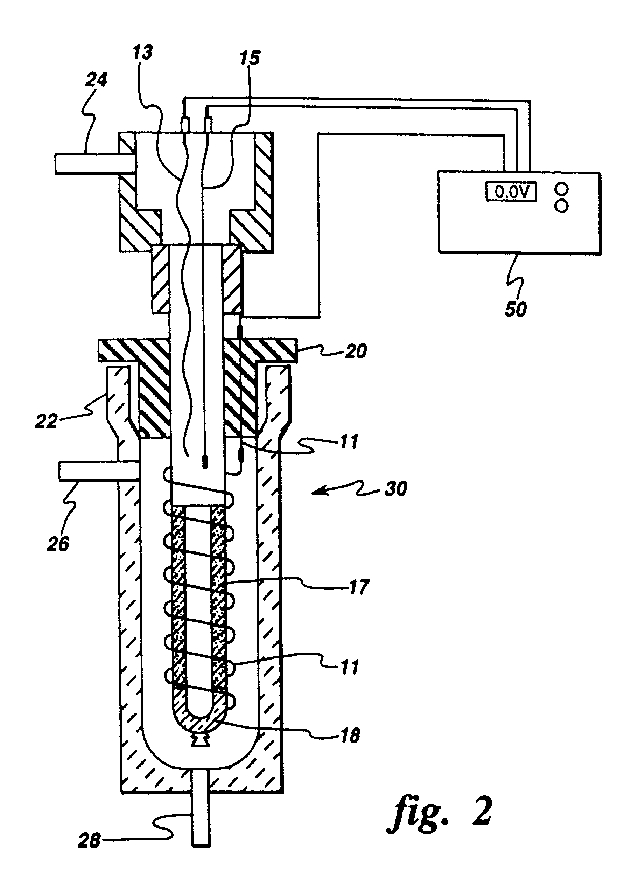

Sensor cell 30 contains three electrodes which are further described in Figure 2. The electrodes are electrically connected to a potentiostat 50, which is electrically connected to a current isolator 52 and data recorder 54.

A thermal enclosure 41 contains a portion of the system including the flow meters 10, 20 and 28 as well as sensor cell 30. The thermal enclosure is provided with a temperature controller 42, one or more heaters 44 and one or more circulation fans 46.

The chlorine monitoring sensor of this invention has a very good linear response to chlorine concentration as shown in Table 1 below.

**TABLE 1.**

| LOW CHLORINE SENSOR CALIBRATION TABLE | |
|---|---|
| CHLORINE CONCENTRATION (ppm) | SENSOR CURRENT (microamp.) |
| 5.2 | 38.13 |
| 4.0 | 27.81 |
| 2.1 | 12.03 |
| 0.7 | 4.19 |
| 0.0 | -0.06 |

**TABLE 2.**

| HIGH CHLORINE SENSOR CALIBRATION TABLE | |
|---|---|
| CHLORINE CONCENTRATION (ppm) | SENSOR CURRENT (microamp.) |
| 0.0 | 0.0 |
| 20.0 | 75.0 |
| 28.3 | 125.0 |
| 43.5 | 200.0 |
| 46.6 | 181.3 |
| 58.5 | 240.6 |
| 88.2 | 428.1 |
| 117.4 | 540.6 |
| 153.0 | 793.8 |
| 180.2 | 1068.8 |
| 215.2 | 1162.5 |
| 224.5 | 1331.3 |
| 293.6 | 1475.0 |

The sensor of this invention possesses great sensitivity, as shown in Table 1, but also has a wide dynamic response range, as shown by data in Table 2. The electrode current is the true current drawn from the working electrode that results from reduction of chlorine. The data clearly show there is a good linear response between the sensors signal and chlorine concentration from 0 to 300 ppm. This data was collected at a flow rate of about 150 cu. cm. per minute. Further experimental results revealed that the linear relationship was even better at higher phosgene flow rates. The experimental conditions include a platinum working electrode, a silver/silver chloride reference electrode and an auxiliary silver electrode. The electrolyte was 0.6 molar lithium chloride at a flow rate of about 5 milliliters per hour. The phosgene flow rate was 150 cubic centimeters per minute and the temperature was maintained at 40°C and applied potential of 0 volts. A preferred operating potential for chlorine reduction is from about -0.1 volt to about +0. volt. At higher potentials the surface of the platinum electrode is oxidized and becomes less effective for chlorine detection.

Figure 2 shows the cell 30 of Figure 1. The electrochemical sensor system comprises an electrochemical cell 30 with at least two electrodes, three as illustrated, a potentiostat or ampmeter, and an electrolyte delivery apparatus, not shown. The most important component of the sensor is the platinum sensing electrode 11, also called the working electrode. Chlorine is electrochemically reduced to chlorine anion (Cl⁻) at the working electrode and the resulting reduction current that passes through the working electrode is detected by an internal measuring device in the potentiostat corresponding to recorder 54 of Fig. 1. External devices can be used. The higher the chlorine concentration in the gas stream, the higher the measured current. Auxiliary electrode 13, also termed a counter electrode is a piece of silver wire. When chlorine is reduced at the working electrode, the silver auxiliary electrode is simultaneously oxidized to silver chloride (AgCl) in an electrolyte containing chlorine anions. The net cell reaction is chlorine oxidizing silver to form silver chloride and no soluble by-product is generated. The third electrode is the reference electrode 15, the function of which is to allow a potentiostat 50 to provide a constant potential for the working electrode. The reference electrode is a silver/silver chloride electrode which can be made by simply inserting a piece of silver wire into an electrolyte containing Cl⁻ anions to form a thin layer of silver chloride at the silver surface. Working electrode 11 is a piece of platinum wire wound around a fritted glass tube 17 of suitable porosity which allows uniform flow of electrolyte. As shown tube 17 has a nonporous lower portion 18 which facilitates flow and drop formation. Cell cap 20 can be sealed in place in the neck of cell body 22 by any convenient means including o-rings or cement. Electrolyte enters the cell via conduit 24. Phosgene and any other gas enters the cell by conduit 26. Gas and electrolyte exit the cell by conduit 28.

## Claims

1. An apparatus for detecting and measuring small amounts of chlorine in phosgene gas comprising a gas delivery system and electrochemical sensor, a temperature control system, a signal measuring system for measuring the current produced by the electrochemical sensor, and optionally a scrubber system for removing chlorine and phosgene from the sensor effluent.

2. An apparatus according to claim 1 in which the gas delivery system comprises an inert gas delivery means, and chlorine standard mixture delivery means, and phosgene delivery means, each of said means having separate flow control devices, metering devices, and check valves.

3. An apparatus according to claim 1 in which the electrochemical sensor comprises a working electrode for electrochemical reduction of chlorine to chloride, a silver auxiliary electrode, a chloride electrolyte, a chloride electrolyte delivery system for continuous delivery of electrolyte to the sensor, both electrodes being in contact with the electrolyte, a potentiostat supplying current to the working electrode, and conduit means for removal of electrolyte from the sensor.

4. An apparatus according to claim 1 in which the electrochemical sensor comprises working electrode for electrochemical reduction of chlorine to chloride, a silver auxiliary electrode, a reference electrode a chloride containing electrolyte, a chloride, electrolyte delivery system for continuous delivery of electrolyte to the sensor, both electrodes being in contact with the electrolyte, a reference electrode to enable the potentiostat to supply a constant potential to the working electrode, and conduit means for removal of electrolyte from the sensor.

5. An apparatus according to claim 4 comprising:
a cell body having gas inlet means, in an upper region thereof, gas and electrolyte outlet means at the bottom of the cell body, an electrode assembly comprising a glass tube connecting with the electrolyte delivery system and having the reference electrode and the auxiliary electrode positioned therein, the glass tube having a porous region and a lower nonporous region, the working electrode being wound around the outer surface of at least the porous region of the glass tube for contact with electrolyte flowing through the porous region.

6. An apparatus according to claim 5 in which the electrode assembly comprises a fritted glass tube having a coating of polytetrafluoroethylene on the outer surface thereof.

7. An apparatus according to claim 1 in which the electrochemical sensor is positioned in a temperature controlled enclosure having heating means and heat distribution means to maintain constant temperature and prevent condensation of phosgene.
